# EUROPEAN PATENT SPECIFICATION

(11) **EP 0 592 504 B1**
(45) Date of publication and mention of the grant of the patent: **02.01.1997**
(21) Application number: 92913728.9
(22) Date of filing: 02.07.1992
(51) Int. Cl.: F24J 1/00, B09B 1/00, A01K 61/00

(54) **IMPROVEMENTS IN THE UTILISATION OF REFUSE**
VERWENDUNG VON ABFÄLLEN
UTILISATION DE DECHETS AMELIOREE

(30) Priority: 04.07.1991 GB 9114462
(43) Date of publication of application: 20.04.1994
(73) Proprietor: BIOMASS RECYCLING LTD., Stafford ST16 2RH (GB)
(72) Inventor: MIDGLEY, Timothy, James, Gnosall Stafford ST20 0HF (GB)
(74) Representative: Tribe, Thomas Geoffrey
(86) International application number: GB9201193
(87) International publication number: WO9301456

(56) References cited:
- EP-A- 0 045 114
- DE-A- 3 425 788
- FR-A- 1 017 119
- FR-A- 2 480 916
- FR-A- 2 481 874
- FR-A- 2 488 983
- FR-A- 2 503 334
- US-A- 3 933 628
- US-A- 4 157 958
- PATENT ABSTRACTS OF JAPAN vol. 6, no. 214 (C-131) 27 October 1982 & JP-A-57 117 399 (HARUNO TAKENAKA) 21 July 1982
- PROGRESS IN ENERGY AND COMBUSTION SCIENCE vol. 8, no. 2, 1982, OXFORD GB pages 141 - 148 'production and use of biogas in agriculture'

## Description

This invention is concerned with the utilisation of refuse. The word 'refuse' is broadly used to cover domestic, commercial and industrial (non-hazardous) waste. The refuse should normally contain biodegradable organic material such as paper, food scraps or wood but sewage may be added to enhance biodegradability. The biodegradability of refuse in ordinary land-fill sites owing to the indigenous bacterial population has been recognised.

FR-A-2,503,334 discloses use of exothermically biodegradable material in a large volume container, whereas US-A- 3,933,628 discloses a large volume container which is used in connection with an endothermically biodegradable material. The present invention aims to solve the problem of producing a system which is an improvement over this prior art.

In the liquid container of the present invention a relatively large volume of liquid is warmed by placing it over refuse undergoing biodegradation with the consequent development of heat.

The liquid container for treatment of biodegradable material comprises a quantity of exothermically biodegradable material contained within a containment layer which lies in a cavity in the ground, and installed directly above the containment layer a flexible liquid impervious layer covering said quantity of exothermically biodegradable material and containing a volume of liquid above said liquid impervious layer so as to define a pond, the flexible impervious layer containing the volume of liquid being continuous so as to fully cover the contained quantity of exothermically biodegradable material and enable the weight of the liquid above said layer to press down on the upper surface of the quantity of exothermically biodegradable material.

The gentle warming achieved makes the invention ideal for a water-filled fish tank or pond. It is known that a mildly elevated water temperature increases the rate of growth of fish and other aquatic life enabling an earlier or better than usual harvest.

In a practical realisation of the invention a large excavated cavity is lined, filled with refuse and a tank or pond filled with water placed over the refuse. As biodegradation takes place the water is gently warmed.

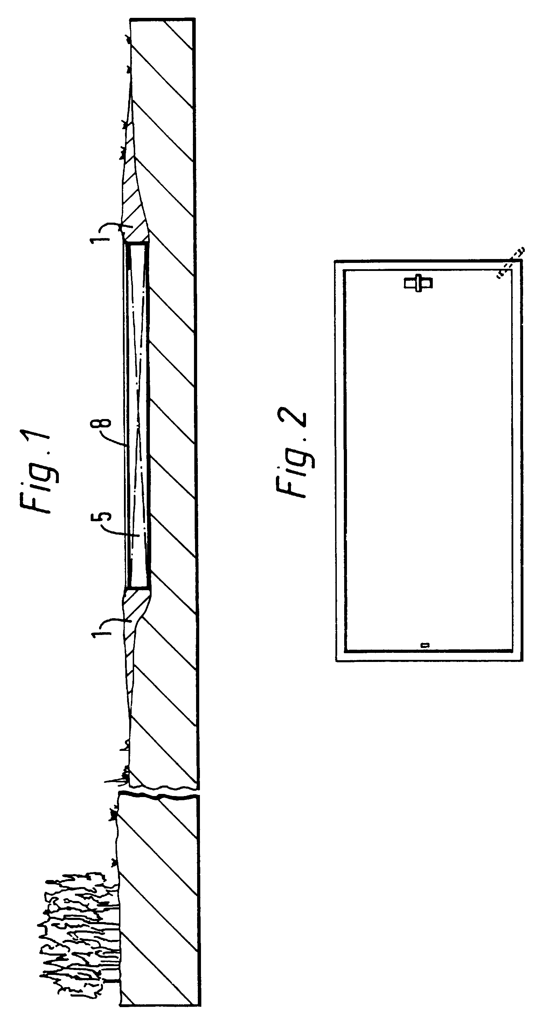

An embodiment of the invention will now be described by way of example and with reference to the accompanying drawings wherein:-
Figure 1 is a longitudinal section through a fish pond in accordance with the invention;
Figure 2 is a plan of the pond at ground level;
Figure 3 is a typical pond section, whilst
Figure 4 is an enlarged view (not to scale) of the excavated and prepared side parts of the pond.

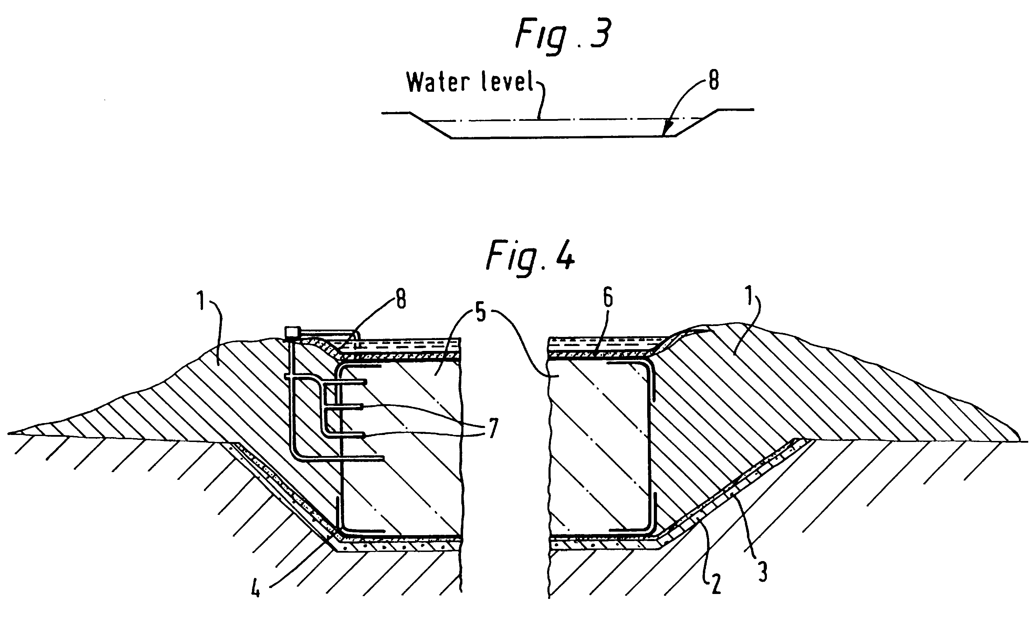

Referring now to the drawings approximately 1 to 3 acres of land are excavated down to approximately 2.5. metres. This area with a pond depth of say one metre (Figure 3) develops a sufficient large volume of water in the sense that it is useful as a fish pond and has a thermal inertia sufficiently high to preclude the possibility of water being warmed to a temperature high enough to harm fish or other aquatic life. The spoil from the excavation is placed around the perimeter to form embankments 1 typically 3.8 metres high. These embankments may be grassed and landscaped. Base layers 2 and 3 of, for example, puddled clay and sand are provided so that an impermeable refuse-containing lining of synthetic flexible membrane 4 for example of butyl rubber or polyethylene of low or high density can be laid without the danger of rupturing in use. The lining 4 which may be double skinned is brought up to define a generally rectangular waste containing space 5 as illustrated and the excavation back-filled to support this space.

This space 5 is then ready to receive refuse and this is tipped in. It is advantageous that the refuse has added to it pressed or filtered sewage sludge to enhance degradation. On completion of the tipping into the space 5 the impermeable membrane is brought over the final surface of the refuse so as to seal in all of the refuse in an airtight manner.

Vent pipes 7 laid into the refuse during deposition are brought through the sides of the liner to a common collection and vent. These pipes 7 serve to collect any gases developed.

The cell is then overlaid for protective purposes with sand or similar at 6 and finally a flexible membrane-lined pond 8 is installed directly over the refuse mass in the space 5. This pond 8 is stocked with fish or other aquatic creatures such as crustacea.

As biodegradation proceeds heat is generated which warms the water in the pond. The whole system may be regarded as a kind of fuel cell.

The methane gas may be used as a fuel for further heating the pool for example or as an alternative to drive an electrical generator.

When anaerobic degradation is complete the pond can be removed and the system opened up. A soil conditioner eemains in the space 5 once residual non-biodegradable materials have been removed. This conditioner is in turn removed for use. The cycle described can then be repeated.

The invention has been described with reference to a particular application, i.e. a fish pond. It has however applicability to any large volume liquid container or reservoir which needs to be warmed gently and could, for example, be used for a temporary swimming pool.

## Claims

1. A large volume liquid container for treatment of biodegradable material comprising a quantity of exothermically biodegradable material contained within a containment layer (4) which lies in a cavity in the ground, and installed directly above the containment layer a flexible liquid impervious layer covering said quantity of exothermically biodegradable material and containing a volume of liquid (8) above said liquid impervious layer so as to define a pond, the flexible impervious layer containing the volume of liquid (8) being continuous so as to fully cover the contained quantity of exothermically biodegradable material and enable the weight of the liquid above said layer to press down on the upper surface of the quantity of exothermically biodegradable material.

2. A large volume liquid container according to claim 1, characterised in that said containment layer (4) comprises at least one synthetic impermeable flexible membrane.

3. A large volume liquid container according to claim 1 or 2, characterised by piping (7) within said biodegradable material for removal of gas.

4. A large volume liquid container according to claim 3, characterised in that said piping (7) for the gas is arranged to direct the gas to a location for collection.

5. A large volume liquid container according to claim 4, characterised in that said collected gas is arranged to be used for additional heating of said volume of liquid.

## Patentansprüche

1. Großvolumiger Flüssigkeitsbehälter zur Behandlung von biologisch abbaubarem Material, mit einer Menge an exothermisch abbaubarem Material, das sich in einer Einschlußschicht (4) befindet, die in einem Hohlraum im Boden liegt, und, direkt über der Einschlußschicht angebracht, einer flexiblen flüssigkeitsundurchlässigen Schicht, die die Menge an exothermisch abbaubarem Material bedeckt und ein Volumen von Flüssigkeit (8) über der flüssigkeitsundurchlässigen Schicht enthält, um einen Teich zu definieren, wobei die flexible, undurchlässige Schicht, die das Volumen von Flüssigkeit (8) enthält, durchgehend ist, so daß sie die eingeschlossene Menge an exothermisch abbaubarem Material vollständig bedeckt und ermöglicht, daß das Gewicht der Flüssigkeit über der Schicht auf die obere Fläche der Menge an exothermisch abbaubarem Material herabdrückt.

2. Großvolumiger Flüssigkeitsbehälter nach Anspruch 1, dadurch gekennzeichnet, daß die Einschlußschicht (4) mindestens eine synthetische, undurchlässige flexible Membran umfaßt.

3. Großvolumiger Flüssigkeitsbehälter nach Anspruch 1 oder 2, gekennzeichnet durch eine Rohrleitung (7) in dem biologisch abbaubaren Material zur Entfernung von Gas.

4. Großvolumiger Flüssigkeitsbehälter nach Anspruch 3, dadurch gekennzeichnet, daß die Rohrleitung (7) für das Gas so angeordnet ist, daß sie das Gas zu einer Sammelstelle leitet.

5. Großvolumiger Flüssigkeitsbehälter nach Anspruch 4, dadurch gekennzeichnet, daß das gesammelte Gas so angeordnet ist, daß es zum zusätzlichen Heizen des Flüssigkeitsvolumens verwendet werden kann.

## Revendications

1. Conteneur de liquide de grand volume pour le traitement de matière biodégradable comprenant une quantité de matière biodégradable exothermiquement contenue au sein d'une couche de confinement (4) qui repose dans une cavité dans le sol, et installée directement au-dessus de la couche de confinement, une couche flexible imperméable aux liquides recouvrant ladite quantité de matière biodégradable exothermiquement et contenant un volume de liquide (8) au-dessus de ladite couche imperméable aux liquides de manière à définir un bassin, la couche imperméable flexible contenant le volume de liquide (8) étant continue de manière à recouvrir entièrement la quantité confinée de matière biodégradable exothermiquement et permettre au poids du liquide au-dessus de ladite couche de presser sur la surface supérieure de la quantité de matière biodégradable exothermiquement.

2. Conteneur de liquide de grand volume selon la revendication 1, caractérisé en ce que ladite couche de confinement (4) comprend au moins une membrane synthétique flexible imperméable.

3. Conteneur de liquide de grand volume selon la revendication 1 ou 2, caractérisé par une tuyauterie (7) à l'intérieur de ladite matière biodégradable pour extraire le gaz.

4. Conteneur de liquide de grand volume selon la revendication 3, caractérisé en ce que ladite tuyauterie (7) pour le gaz est arrangée de manière à diriger le gaz vers une installation de collecte.

5. Conteneur de liquide de grand volume selon la revendication 4, caractérisé en ce que ledit gaz collecté est prévu pour être utilisé pour un chauffage supplémentaire dudit volume de liquide.
